# EUROPEAN PATENT SPECIFICATION

(11) **EP 3 409 319 B1**
(45) Date of publication and mention of the grant of the patent: **18.03.2020**
(21) Application number: 17174191.1
(22) Date of filing: 02.06.2017
(51) Int. Cl.: A61N 1/36, A61N 1/372

(54) **SYSTEM FOR NEURAL HEARING STIMULATION INTEGRATED WITH A PAIR OF GLASSES**
SYSTEM FÜR DIE NEURALE HÖRSTIMULATION INTEGRIERT IN EINER BRILLE
SYSTÈME DE STIMULATION AUDITIVE NEURALE INTÉGRÉ À UNE PAIRE DE LUNETTES

(43) Date of publication of application: 05.12.2018
(73) Proprietor: Advanced Bionics AG, 8712 Staefa (CH)
(72) Inventor: JIANG, Dan, Sevenoaks, Kent TN13 2LL (GB); BOYLE, Patrick J, Beckenham, Kent BR3 1LZ (GB)
(74) Representative: Schwan Schorer & Partner mbB

(56) References cited:
- EP-A1- 0 349 599
- WO-A1-2015/017007
- US-A1- 2012 041 515
- US-A1- 2016 375 242
- US-B1- 8 352 046
- US-B1- 8 583 247

## Description

The invention relates to a system for neural hearing stimulation, comprising an external subsystem, such as a BTE (behind-the-ear) sound processor with a headpiece, and an implantable subsystem, such as a cochlear implant.

Since neural stimulation systems typically comprise both implanted parts and non-implanted parts, transcutaneous transmission of data and power is required, which typically occurs via a radio frequency link between an external coil and an implanted coil which have to be co-located. The required alignment of the coils typically is achieved by a pair of magnets provided at the center of each coil, so that the implanted magnet actually supports the external transmitter coil, which typically is provided as part of a headpiece which is separate from the BTE sound processor (or a body-worn sound processor).

However, implanted magnets may cause problems when the patient has to undergo an MRI scan, since on the one hand, implanted magnets may obscure important parts of the brain, brain stem and cranial nerves from MRI examination and, on the other hand, may result in intolerable pain due to torque forces created by the high magnetic field gradients in MRI scanning devices.

Further, BTE sound processors typically are relatively large in size, so that the pressures applied to the pinna by the BTE housing may cause discomfort and distortion of the external ear. It is also quite difficult to hold the equipment in place in such a manner that a consistent microphone position is achieved, with the microphone position even being more critical for sound processors using beamformers.

In bilateral systems, wherein neural stimulation is provided to both ears or wherein neural stimulation is provided to one ear and acoustic stimulation is provided to the other ear, it is attractive to exchange data between the two sides of the head, with such date exchange typically being implemented by a wireless link. However, wireless transmission requires additional power, is subject to interference or signal loss and imposes some distortion and time delay on shared signals.

It is known to provide the external coil of a partially implantable auditory prosthesis as a part of an eyeglasses frame in the rear part of the arms of the eyeglasses. Such systems are mentioned, for example, in US 4,696,287, US 2013/0110198 A1 and US 6,275,736 B1.

US 2007/0053534 A1 relates to a partially implantable cochlear implant system, wherein the external coil may be fixed to an arm of glasses.

DE 20310025 U1 relates to a headset which may carry the external coil of a cochlear implant.

US 8,352,046 B1 and US 8,977,369 B1 relate to a bilateral cochlear implant system, wherein a microphone and an external transmission coil are attached at each side to the rear end of the respective arm of eyeglasses and wherein a speech processor worn at a rear part of the neck is connected by cables to the microphones and the external transmission coils on both sides.

WO 2015/017007 A1 and US 9,264,824 B2 relate to the use of a hearing device, such as a cochlea implant, with smart glasses including a microphone. The smart glasses may be used for audio and video analysis of a speaker perceived by the user of the smart glasses and the hearing device, wherein the speaker may be identified, voice activity may be detected by analysis of lip movement of the speaker and text generated by speech recognition may be displayed by the smart glasses to the user.

WO 88/09105 A1 relates to a binaural hearing aid system, wherein a hearing aid is provided at each ear, which is mounted at the end of the respective temple piece of eye glasses, wherein the two hearing aids are connected by wires extending through the frame of the eye glasses.

It is an object of the invention to provide for a neural hearing stimulation system, which allows for convenient use by the patient.

According to the invention, this object is achieved by a neural hearing stimulation system as defined in claim 1.

The invention is beneficial in that, by integrating the components of the external subsystem into a pair of eyeglasses, high wearing comfort can be achieved. In particular, compared to conventional systems such as a BTE sound processor, the glasses frame allows for better weight balance, placement of the microphone is more stable in a glasses frame, and the pressure on the pinna or skin flap over the implant site is reduced. Further, the need for an implanted magnet for alignment of the external coil is eliminated by placement of the coil in a glasses arm, thereby improving compatibility with MRI scanning devices, so that the implant patient may undergo MRI scanning under convenient and safe conditions.

Moreover, omission of the magnet in the implant enables development of a substantially smaller implant. Not only could a relatively bulky magnet be avoided, but also the shape of the implanted coil and the location of the implant can be optimized to facilitate the transmission with an implant optimized for use with eyeglasses the external coil and the implanted coil could be greatly simplified. Avoiding the need for an implanted magnet will also improve power efficiency. Different transmission frequencies may be selected for a "break" from full backwards compatibility. The location of the coils can be much lower and behind and above the pinna, thereby greatly improving the cosmetic impact of the system. For the new implant position, surgery, in particular the drilling and flap elevation, is much reduced, and the pressure on the skin flap can be reduced. Also the forces applied whenever the external components of the system are put in place or are removed can be reduced, since in the absence of magnets removal of the external coil does not require overcoming magnetic attraction forces. Optimally, in combination with robotic guided drilling, implantation may be enabled by local anesthetic based day surgery, thereby reducing costs, increasing the number of cases that can be treated and enabling implantation also for patients which are not able to safely undergo general anesthetic.

Placement of forward-facing microphones in the front part of the glasses frame provides for improved noise reduction potential due to the large available space across the face of the glasses frame which allows multiple microphones to be fitted on higher specification versions. In addition, head shadow effects can be reduced compared to BTE sound processors, thereby enabling the microphones to sense a much cleaner sample of the auditory environment, so that beamformer performance can be improved. In addition, the well-defined location of the glasses frame is much less subject to angular movement compared to BTE sound processors, thereby rendering directing of the beamformers more appropriate.

For bilateral or bimodal systems, the glasses frame allows for a relatively simple connection of the two head sides by wire connections provided in the front part of the glasses frame, thereby eliminating the need for a complexity adding and energy consuming wireless connection.

Further preferred embodiments of the invention are defined in the dependent claims.

Hereinafter, examples of the invention will be illustrated by reference to the attached drawings, wherein:
- Fig. 1: is a schematic illustration of an example of the external components of a neural hearing stimulation system according to the invention;
- Fig. 2: is a schematic illustration of the implantable components of a neural hearing stimulation system according to the invention;
- Fig. 3: is a block diagram of an example of a neural hearing stimulation system according to the invention; and
- Fig. 4: is a block diagram of the signal processing in the system of Fig. 3.

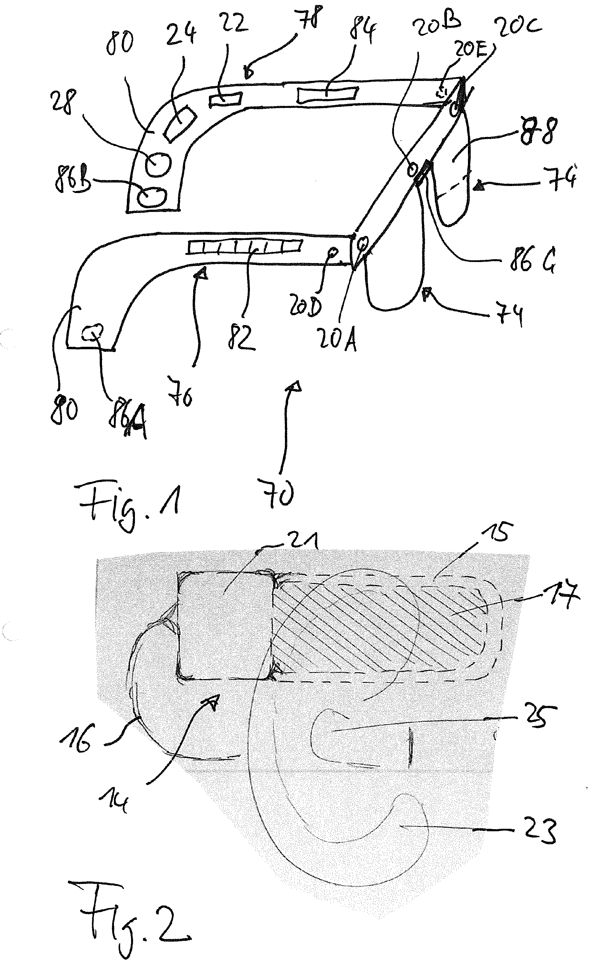

In Figs. 1 to 4 an example of a CI (cochlear implant) system is shown schematically. The system comprises an external sound processing sub-system 10 and an implantable stimulation sub-system 12. The sound processing sub-system 10 serves to detect or sense an audio signal and divide the audio signal into a plurality of analysis channels each containing a frequency domain signal (or simply "signal") representative of a distinct frequency portion of the captured audio. A signal level value is determined for each analysis channel by analyzing the respective frequency domain signal. Stimulation parameters are generated based on the frequency domain signal and are transmitted to the stimulation sub-system 12.

Stimulation sub-system 12 serves to generate and apply electrical stimulation (also referred to herein as "stimulation current" and/or "stimulation pulses") to stimulation sites at the auditory nerve within the cochlea of a patient in accordance with the stimulation parameters received from the sound processing sub-system 10. Electrical stimulation is provided to the patient via a CI stimulation assembly 18 comprising a plurality of stimulation channels.

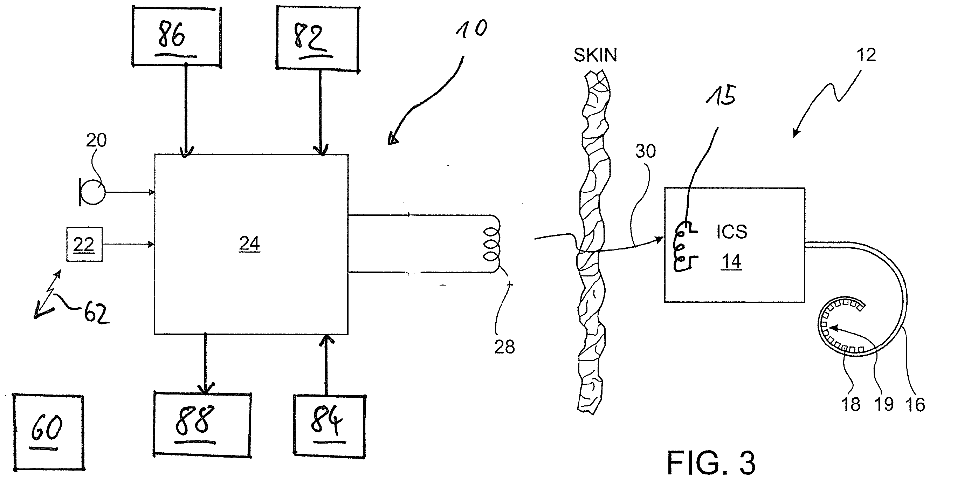

In the example shown in Fig. 3, the stimulation sub-system 12 comprises an ICS (implantable cochlear stimulator) 14, a lead 16 and the stimulation assembly 18 disposed on the lead 16. The stimulation assembly 18 comprises a plurality of stimulation contacts 19 for electrical stimulation of the auditory nerve. The stimulation assembly 18 may be inserted within a duct of the cochlea in such a manner that the stimulation contacts 19 are in communication with one or more stimulation sites within the cochlea, i.e. the stimulation contacts 19 are adjacent to, in the general vicinity of, in close proximity to, directly next to, or directly on the respective stimulation site.

In the example shown in Fig. 3, the sound processing sub-system 10 comprises a microphone arrangement 20 including at least one microphone 20A, 20B for capturing audio signals from ambient sound, a wireless interface 22 for communicating with an external device 60, which may be a media device or a communication device, such as a phone device, a smartphone, a tablet, a media streamer or a remote control, via a wireless link 62, a sound processor unit 24 which receives audio signals from the microphone 20 and the wireless interface 22 and a coil 28. The sound processor unit 24 is configured to process the captured audio signals in accordance with a selected sound processing strategy to generate appropriate stimulation parameters for controlling the ICS 14. In the example of Fig. 3 the sound processor unit 24 is configured to transcutaneously transmit data (in particular data representative of one or more stimulation parameters) to the ICS 14 via a wireless transcutaneous communication link 30. The coil 28 is communicatively coupled to the corresponding coil 15 included within the ICS 14 in order to establish the link 30. The link 30 may include a bidirectional communication link and/or one or more dedicated unidirectional communication links.

The sound processor unit 24 is also provided for controlling operation of the components of the external subsystem 10.

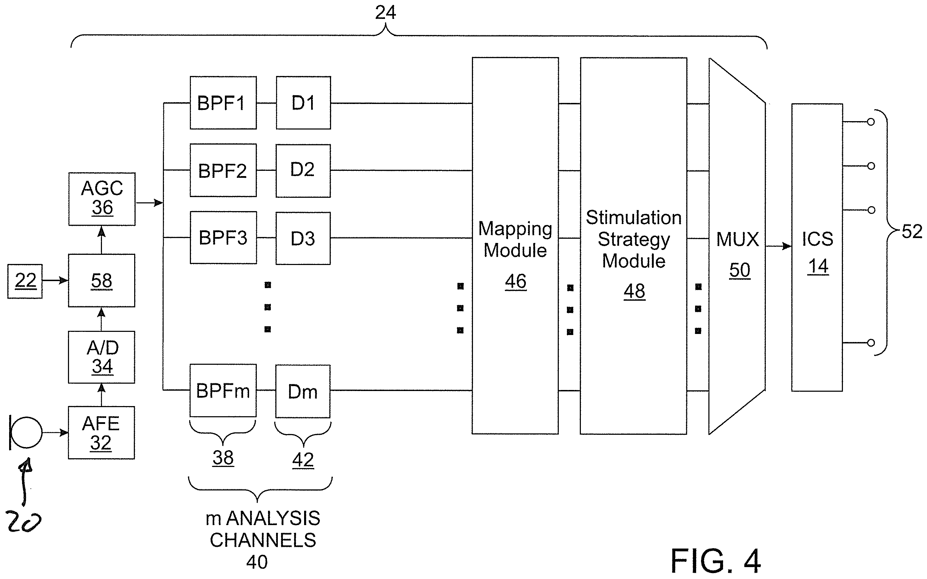

In Fig. 4 a schematic example of the signal processing in a sound processor unit 24 is shown. The audio signals captured by the microphone arrangement 20 are amplified in an audio front end circuitry 32, with the amplified audio signal being converted to a digital signal by an analog-to-digital converter 34. Both the signal from the microphone arrangement 20 and the demodulated audio signal received via the wireless interface 22 are supplied as input signals to a unit 58 which generates a single output signal from these two input signals, which output signal is supplied to an automatic gain control (AGC) unit 36. The AGC function 36 may be implemented as a single channel supplying all of channels that follow, or be realized as a number of separate AGC functions, each dealing with a sub-set of the following channels.

After appropriate automatic gain control, the digital signal is subjected to a filterbank 38 comprising a plurality of filters BPF1 ... BPFm (for example, band-pass filters) which are configured to divide the digital signal into m analysis channels 40, each containing a signal representative of a distinct frequency portion of the audio signal sensed by the microphone 20. For example, such frequency filtering may be implemented by applying a Discrete Fourier Transform to the audio signal and then arranging the resulting frequency bins into the analysis channels 40.

The signals within each analysis channel 40 are input into an envelope detector 42 in order to determine the amount of energy contained within each of the signals within the analysis channels 40 The output signals of the envelope detectors 42 are supplied to a mapping module 46 which serves to map the signals in the analysis channels 40 to the stimulation channels 52. For example, signal levels may be mapped to amplitude values used to define the electrical stimulation pulses that are applied to the patient by the ICS 14 via M stimulation channels 52. For example, each of the m stimulation channels 52 may be associated to one of the stimulation contacts 19 (Fig. 3) or to a group of the stimulation contacts 19.

The sound processor unit 24 further comprises a stimulation strategy module 48 which serves to generate one or more stimulation parameters based on the signals in the analysis channels 40 and in accordance with a certain stimulation strategy (which may be selected from a plurality of stimulation strategies). For example, stimulation strategy module 48 may generate stimulation parameters which direct the ICS 14 to generate and concurrently apply weighted stimulation currents via a plurality 52 of the stimulation channels S*1* ... S*n* in order to effectuate a current steering stimulation strategy. Additionally, or alternatively, the stimulation strategy module 48 may be configured to generate stimulation parameters which direct the ICS 14 to apply electrical stimulation via only a subset N of the stimulation channels 52 in order to effectuate an N-of-M stimulation strategy.

The sound processor unit 24 also comprises a multiplexer 50 which serves to serialize the stimulation parameters generated by the stimulation strategy module 48 so that they can be transmitted to the ICS 14 via the communication link 30 (Fig. 3), i.e. via the coil 28.

The components of the external subsystem 10 are integrated within a pair of eye glasses 70 (Fig. 1), comprising a frame 72 with two lenses 74 and two arms 76, 78 attached to the frame 72 and having a free end portion 80 for supporting the respective arm 76, 78 at the respective ear of the patient. The microphones of the microphone arrangement 20 are integrated as forward facing microphones within the frame 72 (which forms the front-side of the glasses 70), with three microphones 20A, 20B, 20C being illustrated in Fig. 1. Such placement of the microphone arrangement 20 allows for superior noise reduction by acoustic beam forming and by reduction of head shadow effects, with a very stable positioning of the microphone arrangement 20 relative to the patient's head beam being provided by the glasses. While the forward facing microphones 20A, 20B, 20C allow the patient to hear best what he is presently looking at, additional microphones 20D, 20E may be added at the outer side of the arms 76, 78 to detect sounds coming from the sides, which also would help in noise reduction.

User control elements 82 may be located at the outer side of at least one of the arms 76, 78 for enabling convenient user control of the sound processor unit 24. Such positioning allows for relatively large and easily accessible control elements, compared e.g. to control elements on a BTE sound processor housing, which is particularly useful for patients with limited dexterity. Further, since adjusting eyeglasses is a very common and socially perfectly acceptable gesture, the proposed placement of the control elements at the outer side of an eye glasses arm would make adjustment of the auditory prosthesis by the patient more socially acceptable, compared to adjustment of, for example, a BTE sound processor.

The arms 76, 78 also may be used for carrying the power source / battery 84 of the system.

The sound processor unit 24 may be located in one of or both of the arms 76, 78, in particular the rear part 80 of the arms.

The wireless interface 22, including its antenna, may be located in one of the arms 76, 78, with the antenna extending along the respective arm.

In general, the components of the external subsystem 10, like the user control elements 82, the battery 84 and the sound processor unit 24, may be provided only in one of the arms 76, 78 or they may be distributed into both arms 76, 78. In the case of a bilateral or a bimodal system, appropriate sound processors 24 may be housed separately in both arms 76, 78.

The transmission coil 28 for an ICS 14 is provided at one of the arms 76, 78, for example at the rear end 80 of the respective arm, so as to be positioned at the skin close to the implanted coil 15 of the ICS 14. According to one example, the external coil 28, when located in the rear end 80 of the arm 78 may be significantly smaller than a typical headpiece coil. According to another example, the external coil 28 may be a longer coil arranged along the arm 78. As can be seen in Fig. 2, which illustrates the implant position of the ICS 14 with reference to the pinna 23 and the location of the ear canal 25, the implant site of the ICS 14 would be moved compared to conventional systems including a headpiece, with the implanted coil 15 being larger than the external coil 28, so that the external coil 28 for any conceivable use position of the glasses 70 is always within the boundary of the implanted coil 15, with the implanted coil laying underneath where the glasses arm 78 will be when the patient is wearing the glasses 70. As shown in Fig. 2, the ICS 14 includes the implanted coil 15, a silicone support 17 for the coil 15, an implant body 21 and the electrode lead 16.

It is to be noted that typically neither the external subsystem 10 nor the implanted subsystem 12 includes a fixation magnet for aligning the coils 28 and 15. Rather, the position of the external coil 28 is determined by the glasses 70 when worn by the patient.

The external subsystem 10 may include a plurality of skin electrodes 86 (shown as electrodes 86A, 86B and 86C in Fig. 1) which may be used for obtaining electroencephalogram (EEG) signals which may be used for different purposes as will be explained in more detail below. According to the example shown in Fig. 1, each of the rear ends 80 of the arms 76 and 78 may be provided with a skin electrode 86A, 86B, and the frame 72 may be provided with a skin electrode 86C at a position between the two lenses 74, so that the mastoids and the bridge of the nose may be provided with a skin electrode 86 for EEG recordings.

At least one of the lenses 74 may be provided with a head-up display 88 for displaying information to the patient wearing the glasses 70.

According to one example, the skin electrodes 86 may support, for example, electrically evoked brain stem responses (eABR), electrically evoked stapedius reflex threshold (eSRT) or cortical responses. The skin electrodes 88 could support recording ease of listening by the patient to the stimulation provided by the ICS 14, so that an objective evaluation of various sound coding strategies, or parameter sets within a single strategy, may be enabled. The input signal used in such evaluation may be natural everyday sounds where the signal-to-noise ratio (SNR) at the microphone arrangement 20 is monitored, or pre-recorded stimuli delivered by the sound processor unit 24 when such a mode was triggered by the patient.

According to one example, the recorded EEG signals are analyzed by the sound processor unit 24. However, according to an alternative example, analysis would also be possible by use of a smart phone or tablet system wirelessly connected to the glasses 70.

Such detection of listening effort via EEG signals recorded via the electrode 86 may be used to control the signal processing in the sound processor unit 24 accordingly, for example, by activating a beamformer and/or applying a noise reduction algorithm when the sound processor unit 24 judges from an analysis of the recorded EEG signals that the listening effort presently is increasing.

Further, analysis of the recorded EEG signals may be used not only in everyday use of the system, but also during a training mode making the patient familiar with the neural hearing stimulation system. In such user training detection of the listening effort by analyzing recorded EEG signals may be used to determine the training status of the patient, so as to adapt the training program/material accordingly in order to optimize the individual training.

Moreover, the recording of EEG signals via the skin electrodes 86 may be used for controlling certain functions directly by brain activity of the patient. For example, the patient may switch hearing programs or adjust the volume by brain activity, thereby eliminating the need for corresponding manual activity on a mechanical control element. In this regard, such device control by brain activity could be supported by the head-up display 88 which may display to the patient the presently selected hearing program and/or the presently selected value of, for example the volume or sensitivity controls, thereby providing confirmation to the patient that the desired control is actually achieved.

According to another example, the head-up display 88 may be used to support the patient's speech perception by displaying text and/or signs related to what is presently been said. In everyday use of the system, such speech information may be obtained by the system by speech recognition resulting from analysis of the input audio signal. In a training mode, such text information may be provided by the training program running on the sound processor unit 24; for example, speech signals may be provided to the patient via stimulation by the ICS 14, while the corresponding text is presented to the patient via the head-up display 88. Further, the training material may include video material which may be presented to the patient via the head-up display 88 during stimulation by the ICS 14; for example, the video could show speaking person while the respective speech is presented to the patient via the ICS 14 in a synchronized manner.

Alternatively or in addition, a media device (acting as the external device 60), such as a smartphone or a tablet, may be involved for providing training material to the patient, such as video material.

Further, in a training mode the head-up display 88 may be used for providing instructions to the patient. Training programs may, for example, play pre-recorded speech with various amounts of competing noise, with the patient responding to the correct visual representation provided by the head-up display 88 by manually acting on the user control elements 82 provided at the arms 76, 78.

In everyday use of the system, the head-up display 88 could be used to inform the patient of the present settings of the sound processor unit 24, such as the volume, the hearing program, etc. In addition, the head-up display 88 could be used for informing the patient regarding the present use situation, such as the auditory scene / acoustic conditions as presently detected by the sound processor unit 24 by analyzing the audio input signal, quality of the input audio signal, recommendation by the sound processor unit 24 to switch to another program which is considered to be more appropriate for the present acoustic conditions, etc. Moreover, the head-up display 88 could be used to inform the patient concerning the status of the system, such as battery condition, detection of degradation of certain components, such as microphones and cables, an alert that a call is coming in from a mobile phone, etc., including warnings in case that a detected condition of the system is not acceptable.

The present invention may be used not only for mono-lateral systems, but also for bilateral implants or for bimodal systems (i.e. systems including one neural implant and one acoustic hearing aid). For a bilateral implant, both arms 76, 78 would be provided, for example at their rear end 80, with an external coil 28.

Both in mono-lateral and bilateral/bimodal systems a wire connection may extend through the front frame part 72 so as to electrically connect the components provided in the two arms 76, 78, without the need for a wireless connection.

For bilaterally implanted patients a training may be particularly useful in case that first only one ear is implanted and later the second ear is implanted. In such training, the sound delivered to each implant could be varied adaptively so that for pre-programmed or automatically detected times, the first implanted side is provided with less clear sound, thereby forcing the brain to pay more attention to the newer implant, while bilateral listening still would be promoted, resulting in a particularly rapid optimization of bilateral listening.

Further, a self-programing system could be easily setup as a standalone device by displaying instructions of targets on the head-up display 88, while parameters are varied to produce various stimulation patterns which are to be optimized; such system also may include objective measurements recorded from within the cochlea, including those arising from either electrical or acoustic stimulation, for example, electrically evoked compound action potentials (eCAPs), or electrocochleography (ECochG).

## Claims

1. A system for neural hearing stimulation, comprising an external sub-system (10) and an implantable sub-system (12),
the external sub-system comprising a microphone arrangement (20) for capturing an input audio signal, a sound processor unit (24) for generating a processed signal from the input audio signal and for controlling the external subsystem, and an external coil (28) for establishing a transcutaneous link (30) for transmitting the processed signal to the implantable sub-system;
the implantable sub-system comprising an implantable coil (15) for receiving the processed signal, a unit (14) for generating a neural stimulation signal from the processed signal, and a unit (16, 18, 19) for neural stimulation of the patient's hearing according to the neural stimulation signal,
wherein the external sub-system comprises a pair of eyeglasses (70), wherein the external coil is located in one of the arms of the glasses, **characterized in that** the pair of eyeglasses (70) includes a user interface (82) for manual user control of the sound processor unit and a battery (84), wherein at least one microphone (20A, 20B, 20C) of the microphone arrangement is located at the front-side of the frame (72) of the glasses and the sound processor unit is located in at least one of the arms (76, 78) of the glasses.

2. The system of claim 1, wherein the external coil (28) and/or the sound processor unit (24) is located in a free end portion (80) of at least one of the glasses arms (76, 78).

3. The system of one of the preceding claims, wherein the user interface comprises control elements (82) located at an outwardly facing side of at least one of the glasses arms (76).

4. The system of one of the preceding claims, wherein the battery (84) is located in at least one of the glasses arms (76, 78).

5. The system of one of the preceding claims, wherein the external subsystem (10) comprises a plurality of skin electrodes (86) configured to touch the patient's skin, with the skin electrodes being supported by the glasses (70), wherein the system is configured to record EEG signals via the skin electrodes (86), in particular in response to at least one of: hearing stimulation via the implantable subsystem and presentation of information to the patient via a head-up display in at least one of the spectacles lenses, and wherein each of the glasses arms (76, 78) and/or the front part of the glasses frame (72) comprises at least one of the skin electrodes (86A, 86B, 86C).

6. The system of claim 5, wherein the sound processor unit (24) is configured to analyze the recorded EEG signals and to use the results of the analysis for control of the signal processing, in particular for controlling at least one of volume adjustment, stimulation mode selection, acoustic beamforming, noise reduction, and adjustment of training programs.

7. The system of one of claims 1 to 4, wherein at least one of the lenses (74) of the glasses (70) comprises a head-up display (88) for displaying information to the patient.

8. The system of one of the preceding claims, wherein the external sub-system (10) comprises a wireless interface (22) to an external communication device (60), with the wireless interface being integrated within the glasses (70), and wherein an antenna of the wireless interface is arranged within one of the arms (78) of the glasses (70).

9. The system of one of the preceding claims, wherein the implantable subsystem (12) comprises a cochlear implant (14, 16, 18, 19) including a cochlear electrode array (19).

10. The system of one of the preceding claims, wherein the system is configured to apply hearing stimulation to a single one of the patient's ears, wherein the user interface (82), the sound processor unit (24), the battery (84) and the external coil (28) are distributed onto both arms (76, 78) of the glasses (70), and wherein the arms are electrically connected by wires running through the front part of the glasses frame (72).

11. The system of one of claims 1 to 9, wherein the system is configured to apply synchronized hearing stimulation to both ears of the patient, wherein the implantable sub-system (12) comprises the implantable coil (15), the neural stimulation signal generating unit (14) and the neural stimulation unit (16, 18, 19) at one side of the patient's head and another implantable coil (15), another neural stimulation signal generating unit (14) and another neural stimulation unit (16, 18, 19) at the other side of the patient's head, wherein one of the arms (76) of the glasses (70) comprises the external coil (28) and the sound processor unit (24) dedicated to the part of the implantable sub-system to be implanted in the respective side of the patient's head, wherein the other one of the arms (78) of the glasses (70) comprises another external coil (28) and another sound processor unit (24) dedicated to the part of the implantable sub-system to be implanted in the other side of the patient's head, and wherein the glasses arms are electrically connected by wires running through the front part of the glasses frame (72).

## Patentansprüche

1. System zur neuralen Gehörstimulation, mit einem externen Untersystem (10) und einem implantierbaren Untersystem (12),
wobei das externe Untersystem eine Mikrofonanordnung (20) zum Auffangen eines Eingangs-Audiosignals, eine Schallverarbeitungseinheit (24) zum Erzeugen eines verarbeiteten Signals aus dem Eingangs-Audiosignal und zum Steuern des externen Untersystems sowie eine externe Spule (28) zum Aufbauen einer transkutanen Verbindung (30) zum Senden des verarbeiteten Signals zu dem implantierbaren Untersystem aufweist;
wobei das implantierbare Untersystem eine implantierbare Spule (15) zum Empfangen des verarbeitenden Signals, eine Einheit (14) zum Erzeugen eines neuralen Stimulationssignals aus dem verarbeiteten Signal sowie eine Einheit (16, 18, 19) zum neuralen Stimulieren des Gehörs des Patienten gemäß dem neuralen Stimulationssignal aufweist,
wobei das externe Untersystem eine Brille (70) aufweist, wobei die externe Spule in einem der Brillenbügel angeordnet ist, **dadurch gekennzeichnet, dass** die Brille (70) eine Benutzerschnittstelle (82) zur manuellen Benutzersteuerung der Schallverarbeitungseinheit und eine Batterie (84) aufweist, wobei mindestens ein Mikrofon (20A, 20B, 20C) der Mikrofonanordnung an der Stirnseite des Rahmes (72) der Brille angeordnet ist und die Schallverarbeitungseinheit in mindestens einem der Bügel (76, 78) der Brille angeordnet ist.

2. System gemäß Anspruch 1, wobei die externe Spule (28) und/oder die Schallverarbeitungseinheit (24) in einem freien Endbereich (80) mindestens eines der Brillenbügel (76, 78) angeordnet ist bzw. sind.

3. System gemäß einem der vorherigen Ansprüche, wobei die Benutzerschnittstelle Steuerelemente (82) aufweist, die auf einer nach außen weisenden Seite mindestens eines der Brillenbügel (76) angeordnet ist.

4. System gemäß einem der vorherigen Ansprüche, wobei die Batterie (84) in mindestens einem der Bügel (76, 78) der Brille angeordnet ist.

5. System gemäß einem der vorherigen Ansprüche, wobei das externe Untersystem (10) eine Mehrzahl von Haut-Elektroden (86) aufweist, die ausgebildet sind, um die Haut des Patienten zu berühren, wobei die Haut-Elektroden von der Brille (76) getragen werden, wobei das System ausgebildet ist, um EEG-Signale über die Haut-Elektroden (86) aufzuzeichnen, insbesondere als Reaktion auf: Gehörstimulation mittels des implantierbaren Untersystems und/oder Darbieten von Information für den Patienten über eine Blickfeldanzeige in mindestens einem der Brillengläser, und wobei jeder der Brillenbügel und/oder der Stirnteile des Brillenrahmens (72) mindestens eine der Haut-Elektroden (86A, 86B, 86C) aufweist bzw. aufweisen.

6. System gemäß Anspruch 5, wobei die Schallverarbeitungseinheit (24) ausgebildet ist, um die aufgezeichneten EEG-Signale zu analysieren und die Ergebnisse der Analyse zum Steuern der Signalverarbeitung zu verwenden, insbesondere zum Steuern der Lautstärkeeinstellung, der Stimulationsmodusauswahl, dem akustischen Beamforming, der Geräuschverringerung, und/oder dem Einstellen von Trainingsprogrammen.

7. System gemäß einem der Ansprüche 1 bis 4, wobei mindestens eines der Gläser (74) der Brille (70) eine Sichtfeldanzeige (88) zum Anzeigen von Informationen für den Patienten aufweist.

8. System gemäß einem der vorhergehenden Ansprüche, wobei das externe Untersystem eine drahtlose Schnittstelle (22) zu einem externen Kommunikationsgerät (60) aufweist, welche in die Brille integriert ist, und wobei eine Antenne der drahtlosen Schnittstelle in einem der Bügel (78) der Brille (70) angeordnet ist.

9. System gemäß einem der vorhergehenden Ansprüche, wobei das implantierbare Untersystem (12) ein Cochlea-Implantat (14, 16, 18, 19) mit einer Cochlea-Elektrodenanordnung (19) aufweist.

10. System gemäß einem der vorhergehenden Ansprüche, wobei das System ausgebildet ist, um Gehörstimulation auf ein einzelnes Ohr des Patienten anzuwenden, wobei die Benutzerschnittstelle (82), die Schallverarbeitungseinheit (24), die Batterie (84) und die externe Spule auf die beiden Bügel (76, 78) der Brille (70) verteilt sind, und wobei die Bügel elektrisch mittels Drähten verbunden sind, die durch den Stirnteil des Brillenrahmens (72) laufen.

11. System gemäß einem der Ansprüche 1 bis 9, wobei das System ausgebildet ist, um synchronisierte Gehörstimulation auf beide Ohren des Patienten anzuwenden, wobei das implantierbare Untersystem (12) die implantierbare Spule (15), die Einheit (14) zum Erzeugen des neuralen Stimulationssignals und die neurale Stimulationseinheit (16, 18, 19) auf einer Seite des Kopfs des Patienten und eine weitere implantierbare Spule (15), eine weitere Neural-Stimulationssignal-Erzeugungseinheit (14) sowie eine weitere Neural-Stimulationseinheit (16, 18, 19) auf der anderen Seite des Kopfs des Patienten aufweist, wobei einer der Bügel (76) der Brille die externe Spule (28) und die Schallverarbeitungseinheit (24) aufweist, welche dem Teil des implantierbaren Untersystems zugeordnet sind, welches auf der jeweiligen Seite des Kopfs des Patienten zu implantieren ist, wobei der andere Bügel (78) der Brille (70) eine weitere externe Spule (28) und eine weitere Schallverarbeitungseinheit (24) aufweist, welche dem Teil des implantierbaren Untersystems zugeordnet sind, welcher auf der anderen Seite des Kopfs des Patienten zu implantieren ist, und wobei die Brillenbügel mittels Drähten elektrisch verbunden sind, die durch den Stirnteil des Brillenrahmens (72) laufen.

## Revendications

1. Système pour une stimulation auditive neuronale, comprenant un sous-système externe (10) et un sous-système implantable (12),
le sous-système externe comprenant un agencement de microphone (20) pour capturer un signal audio d'entrée, une unité de processeur de sons (24) pour générer un signal traité à partir du signal audio d'entrée et pour commander le sous-système externe, et une bobine externe (28) pour établir une liaison transcutanée (30) pour transmettre le signal traité au sous-système implantable ;
le sous-système implantable comprenant une bobine implantable (15) pour recevoir le signal traité, une unité (14) pour générer un signal de stimulation neuronale à partir du signal traité, et une unité (16, 18, 19) pour une stimulation neuronale de l'audition du patient en fonction du signal de stimulation neuronale,
dans lequel le sous-système externe comprend une paire de lunettes (70), dans lequel la bobine externe est située dans une des branches de lunettes, **caractérisé en ce que** la paire de lunettes (70) comprend une interface utilisateur (82) pour une commande utilisateur manuelle de l'unité de processeur de son et une batterie (84), dans lequel au moins un microphone (20A, 20B, 20C) de l'agencement de microphone est situé au niveau du côté avant de la monture (72) de lunettes et l'unité de processeur de sons est située dans au moins une des branches (76, 78) des lunettes.

2. Système selon la revendication 1, dans lequel la bobine externe (28) et/ou l'unité de processeur de sons (24) sont situés dans une partie extrémité libre (80) d'au moins une des branches (76, 78) de lunettes.

3. Système selon l'une des revendications précédentes, dans lequel l'interface utilisateur comprend des éléments de commande (82) situés au niveau d'un côté tourné vers l'extérieur d'au moins une des branches (76) de lunettes.

4. Système selon l'une des revendications précédentes, dans lequel la batterie (84) est située dans au moins une des branches (76, 78) de lunettes.

5. Système selon l'une des revendications précédentes, dans lequel le sous-système (10) comprend une pluralité d'électrodes cutanées (86) conçues pour toucher la peau du patient, les électrodes cutanées étant supportées par les lunettes (70), dans lequel le système est configuré pour enregistrer des signaux EEG par l'intermédiaire des électrodes cutanées (86), en particulier en réponse à au moins un parmi : une stimulation auditive par l'intermédiaire du sous-système implantable et la présentation d'informations au patient par l'intermédiaire d'un affichage tête haute dans au moins un des verres de lunettes, et dans lequel chacune des branches (76, 78) de lunettes et/ou la partie avant de la monture (72) de lunettes comprennent au moins une des électrodes cutanées (86A, 86B, 86C).

6. Système selon la revendication 5, dans lequel l'unité de processeur de sons (24) est configurée pour analyser les signaux EEG enregistrés et pour utiliser les résultats de l'analyse pour la commande du traitement de signaux, en particulier pour commander le réglage du volume, et/ou la sélection du mode de stimulation, et/ou la formation de faisceau acoustique, et/ou la réduction du bruit et/ou l'ajustement de programmes d'apprentissage.

7. Système selon l'une des revendications 1 à 4, dans lequel au moins un des verres (74) des lunettes (70) comprend un affichage tête haute (88) pour afficher des informations au patient.

8. Système selon l'une des revendications précédentes, dans lequel le sous-système externe (10) comprend une interface sans fil (22) avec un dispositif de communication externe (60), l'interface sans fil étant intégrée dans les lunettes (70), et dans lequel une antenne de l'interface sans fil est agencée à l'intérieur de l'une des branches (78) de lunettes (70).

9. Système selon l'une des revendications précédentes, dans lequel le sous-système implantable (12) comprend un implant cochléaire (14, 16, 18, 19) comprenant un réseau d'électrodes cochléaires (19).

10. Système selon l'une des revendications précédentes, dans lequel le système est configuré pour appliquer une stimulation auditive à une seule des oreilles du patient, dans lequel l'interface utilisateur (82), l'unité de processeur de sons (24), la batterie (84) et la bobine externe (28) sont répartis sur les deux branches (76, 78) des lunettes (70), et dans lequel les branches sont connectées électriquement par des fils parcourant la partie avant de la monture (72) de lunettes.

11. Système selon l'une des revendications 1 à 9, dans lequel le système est configuré pour appliquer une stimulation auditive synchronisée aux deux oreilles du patient, dans lequel le sous-système implantable (12) comprend la bobine implantable (15), l'unité de génération de signaux de stimulation neuronale (14) et l'unité de stimulation neuronale (16, 18, 19) sur un côté de la tête du patient et une autre bobine implantable (15), une autre unité de génération de signaux de stimulation neuronale (14) et une autre unité de stimulation neuronale (16, 18, 19) sur l'autre côté de la tête du patient, dans lequel une des branches (76) des lunettes (70) comprend la bobine externe (28) et l'unité de processeur de sons (24) dédiées à la partie du sous-système implantable devant être implanté dans le côté respectif de la tête du patient, dans lequel l'autre des branches (78) des lunettes (70) comprend une autre bobine externe (28) et une autre unité de processeur de sons (24) dédiées à la partie du sous-système implantable devant être implanté dans l'autre côté de la tête du patient, et dans lequel les branches de lunettes sont connectées électriquement par des fils parcourant la partie avant de la monture (72) de lunettes.
